# EUROPEAN PATENT APPLICATION

(11) **EP 1 813 262 A1**
(43) Date of publication of application: **01.08.2007**
(21) Application number: 06001090.7
(22) Date of filing: 19.01.2006
(51) Int. Cl.: A61K 8/81, A61K 8/898, A61K 8/73, A61K 8/41, A61Q 5/12, A61Q 5/00

(54) **Stable oil-in-water and water/oil/water multiple emulsions and hair treating compositions comprising them**

(71) Applicant: Wella Aktiengesellschaft, 64274 Darmstadt (DE)
(72) Inventor: Kolly, Maryline Hernandez, 1762 Givisiez (CH); Müller, Timothy, 64850 Schaafheim (DE); Springob, Christian, Dr., 64653 Lorsch (DE); Mönks, Monika, 3185 Schmitten (CH); Weber, Dirk, Dr., 1723 Marly (CH)

(57) **Abstract**

The present invention provides an oil-in-water or water/oil/water multiple emulsion, or a mixture thereof, comprising
(A) a organopolysiloxane elastomer crosslinked with polyether chains and
(B) at least one thickener selected from the group of a polyacrylamide homo- or copolymer, a celluloseether, a non-silicone cationic polymer or or a thickening system comprising a mixture of from about 60 to about 67% of the thickening system of a copolymer of 2-propenoic acid with 2-propenamide, from about 27 to about 32% of the thickening system of the homopolymer of 2-methyl-1-propene and from about 5 to about 7% of the thickening system of poly(oxy-1,2-ethanediyl)-sorbitan-monododecanoate, and cosmetic compositions comprising this emulsion.

## Description

### TECHNICAL FIELD

The invention relates to a stable oil-in-water (O/W) or water/oil/water (W/O/W) multiple emulsion, or the mixture thereof, and to its use, preferably in the cosmetics field. The emulsion may be used more preferably for treating keratinous fibers and most preferably for compositions for conditioning hair.

### BACKGROUND OF THE INVENTION

It is known, in particular in the cosmetics and dermatological fields, to use topical compositions in the form of emulsions. These emulsions are generally oil-in-water (O/W) or water-in-oil (W/O) emulsions. These compositions may also be multiple emulsions of the water/oil/water (W/O/W) or oil/water/oil (O/W/O) type. Use is preferably made, among multiple emulsions, of emulsions with an aqueous external phase, namely W/O/W emulsions, which combine the advantages of freshness on application, contributed by the water present in the aqueous external phase, and of comfort, contributed by a relatively large amount of oil.

However, multiple emulsions are not exploited to any great extent because they frequently exhibit problems of stability over time.

A variety of approaches have been developed to provide stable emulsions for compositions to condition hair.

US 2002/0159963 A1 describes a W/O/W triple emulsion, comprising an aqueous external phase and a W/O primary emulsion, wherein the primary emulsion comprises an oily phase and an aqueous internal phase, wherein the triple emulsion comprises at least one partially or completely crosslinked organopolysiloxane elastomer comprising a polyoxyethylenated and/or polyoxypropylenated chain. However also these emulsions are not sufficient stable and difficult to manufacture. Triple emulsions of the W/O/W or else the O/W/O type are also used in cosmetics or dermatology. However, such emulsions are subject to numerous problems during their production, or else to problems of stability over time, and in particular when active substances which may have a tendency to destabilize the prepared emulsions are introduced into these emulsions. It is also of great importance, that the emulsion is compatible with other raw materials. The emulsion should further be stable with fatty alcohol and cationic surfactants. Furthermore another requirement of the emulsion is a plain manufacturing procedure. Preferably the multiple emulsion should be formed just by stirring or homogenizing the components it in a one-step-process. While it is prepared by stirring or homogenization it should display no sensitivity against shear stress.

The need therefore remains for a stable O/W or W/O/W multiple emulsion, or the mixture thereof, which does not have the disadvantages of known emulsions and which is, in particular, pleasant to use on the hair or skin while contributing, for example, the advantages of an emulsion with an aqueous external phase. The emulsion as well as the cosmectic compositions comprising it should display a high water content for a good moisturizing effect on skin or hair however without showing a coating effect on hair.

### SUMMARY OF THE INVENTION

The inventors have now unexpectedly found, that the introduction of
(A) a organopolysiloxane elastomer crosslinked with polyether chains preferably with polyoxyethylen, polyoxypropylen and/or polyglycerin chains, in combination with
(B) a thickener selected from the group of a polyacrylamide homo- or copolymer, a celluloseether, such as methyl cellulose, hydroxyethyl cellulose, hydroxymethylcellulose, a cationic polymer, or a thickening system comprising a mixture of from about 60 to about 67% of a copolymer of 2-propenoic acid with 2-propenamide (CAS 26100-47-0), from about 27 to about 32% of the homopolymer of 2-methyl-1-propene (CAS 9003-27-4; CTFA: POLYISOBUTENE), and from about 5 to about 7% of poly(oxy-1,2-ethanediyl)-sorbitan-monododecanoate (CAS 9005-64-5; CTFA: POLYSORBATE-20) into a oil-in-water (O/W) or a water/oil/water (W/O/W) emulsion, makes it possible to stabilize the emulsion with advantages not achieved by the prior art emulsions.

According to this the applicant claims the emulsion according to claim 1 and cosmetic compositions according to claims 13 to 21 comprising said emulsion. Preferred embodiments are disclosed in the dependent claims.

Another aspect of the present invention is the use of (A) a organopolysiloxane elastomer cross linked with polyether chains preferably with polyoxyethylen, polyoxypropylen and/or polyglycerin chains, in combination with (B) a thickener selected from the group of a polyacrylamide homo- or copolymer, a celluloseether, a cationic polymer, or a thickening system comprising a mixture of from about 60 to about 67% of a copolymer of 2-propenoic acid with 2-propenamide (CAS 26100-47-0), from about 27 to about 32% of the homopolymer of 2-methyl-1-propene (CAS 9003-27-4; CTFA: POLYISOBUTENE), and from about 5 to about 7% of poly(oxy-1,2-ethanediyl)-sorbitan-monododecanoate (CAS 9005-64-5; CTFA: POLYSORBATE-20) for the stabilization of a water/oil or water/oil/water multiple emulsion. For topical application, the inventive emulsion may contain a topically acceptable medium, i.e. a medium compatible with the skin, mucous membranes and/or keratinous fibers, such as the hair.

The emulsion according to the invention has the advantage of being stable and of being able in particular to retain the activity of active agents present in the aqueous internal phase, whence they are released during the application of the composition to the skin, mucous membranes and/or hair. It is also of great importance, that the emulsion is compatible with other raw materials such as fatty alcohols and cationic surfactants which on their parts may form a liquid-cristalline-network. Another advantage of the emulsion of the invention is the plain manufacturing procedure. The multiple emulsion is formed just by stirring or homogenizing the oily phase with the aqueous phase, which method delivers multiple emulsions even at very high shear rates.

A more complete appreciation of the invention and many of the attendant advantages thereof will be readily obtained as the same becomes better understood by reference to the following detailed description.

### DETAILED DESCRIPTION OF THE INVENTION

These and other objects and benefits of the present invention as may be set forth herein as may now or later become apparent to those skilled in the art can be provided according to the invention which is described herein.

The invention hereof can comprise, consist of, or consist essentially of the essential elements described herein as well as any of the preferred or other optional ingredients described herein.

All percentages herein are by weight of the composition unless otherwise indicated. All ratios are weight ratios unless otherwise indicated. Unless otherwise indicated, all percentages, ratios, and levels of ingredients referred to herein are based on the actual amount of the ingredient, and do not include solvents, fillers, or other materials with which the ingredient may be combined in commercially available products. All documents referred to herein, including all patents, all patent applications and all articles, are hereby incorporated herein by reference in their entirety.

The present invention also provides a method for conditioning hair by application to the hair of an effective amount of the compositions hereof to enhance glossiness of the hair.

The essential ingredients of the hair treating composition, which preferably is a hair conditioning composition, as well as a variety, but non-exclusive, list of preferred and optional ingredients are described below.

### CROSSLINKED ORGANOPOLYSILOXANE ELASTOMER

From the organopolysiloxane elastomer crosslinked with polyether chains are those preferred which are crosslinked by polyoxyethylen, polyoxypropylen and/or polyglycerin chains, whereby those are most preferred, which are crosslinked by polyglycerin chains.

The organopolysiloxanes of the composition of the invention comprise one or more oxyalkylenated and in particular polyglycerine groups, for example from 1 to 40 oxyalkylenated units and better 1 to 20 oxyalkylenated units, which can form polyoxyalkylene chains. These groups are intended to connect two parts of the silicone structure.

Although to organopolysiloxanes may be crosslinked by oxyethylenated chains or oxypropylenated chains, the invention relates more preferably to organopolysiloxanes crosslinked by polyglycerine chains. The organopolysiloxanes can also simultaneously comprise polyglycerine chains, oxyethylenated (OE) chains, for example 1 to 20, and oxypropylen (OP) chains, for example 0 to 20.

Preferably crosslinked organopolysiloxanes with polyglycerine chains, the products sold by Shin-Etsu Chemical Co. Ltd., Tokyo, Japan under the name of KSG-210, KSG-710, KSG-310, KSG-320, KSG-330, KSG-340, KSG-810, KSG-820, KSG-830 and KSG-840 may be used. The organopolysiloxanes of the invention may be obtained according to the procedure of U.S. Pat. No. 6,784,271 B2 (incorporated herein by reference).

The crosslinked organopolysiloxane elastomer can be contained in the emulsion as well as in the hair treating composition of the invention at levels of from about 0.1 % to about 10% by weight of the composition, preferably from about 0.5% to about 6%, more preferably from about 1 % to about 5%, most preferably from about 2% to about 4 % by weight.

### THICKENERS

### Polyacrylate Homo- or Copolymer Thickeners

A preferred thickener according to the invention is the homopolymerisate of acrylamide. More preferred is a mixture of polyacrylamide, C13-14-isoparaffin, and polyoxyethylene (7) laurylether. Most preferred is a mixture of 35 - 45% by weight of the mixture of polyacrylamide, 15-25 % by weight of the mixture of C13-14-isoparaffin, and 3 to 8% by weight of the mixture polyoxyethylene (7) laurylether which optionally contains water up to 100%. A suitable thickener product is sold under the trade name SEPIGEL 305 by the company Seppic Inc. USA;. A further suitable polymer is Hydroxyethyl acrylate/sodium Acryloyldimethyl taurate copolymer (Simulgel^{®} NS).

The thickening system mentioned in claim 1 preferably comprises a mixture of
(a) from about 60 to about 67% of a copolymer of 2-propenoic acid with 2-propenamide (CAS 26100-47-0);
(b) from about 27 to about 32% of the homopolymer of 2-methyl-1-propene (CAS 9003-27-4; CTFA: POLYISOBUTENE);
(c) from about 5 to about 7% of poly (oxy-1,2-ethanediyl)-sorbitan-monododecanoate (CAS 9005-64-5; CTFA: POLYSORBATE-20) and
(d) from 0 to about 22% by weight of water.

A thickening system according to the invention is sold under the trade name Sepiplus^{®} 265, by SEPPIC Inc. USA; Another but less advantageous thickener is Sepiplus^{®} 400 of SEPPIC Inc. USA.

### Cationic Polymer Thickeners

The compositions of the present invention can also comprise one or more cationic non-silicone polymer as thickening agent. The cationic non-silicone polymer thickening agent will preferably be water soluble.

By "water soluble" cationic non-silicone organic polymer, what is meant is a polymer which is sufficiently soluble in water to form a substantially clear solution to the naked eye at a concentration of 0.1 % in water (distilled or equivalent) at 25°C. Preferably, the polymer will be sufficiently soluble to form a substantially clear solution at 0.5% concentration, more preferably at 1.0% concentration. As used herein, the term "polymer" shall include materials whether made by polymerization of one type of monomer or made by two (i.e., copolymers) or more types of monomers.

The cationic polymers hereof will generally have a weight average molecular weight which is at least about 5,000, typically at least about 10,000, and is less than about 10 million. Preferably, the molecular weight is from about 100,000 to about 2 million. The cationic polymers will generally have cationic nitrogen-containing moieties such as quaternary ammonium or cationic amino moieties, or a mixture thereof.

The cationic charge density is preferably at least about 0.1 meq/gram, more preferably at least about 1.5 meq/gram, even more preferably at least abut 1.1 meq/gram, most preferably at least about 1.2 meq/gram. Cationic charge density of the cationic polymer can be determined according to the Neldahl Method. Those skilled in the art will recognize that the charge density of amino-containing polymers may vary depending upon pH and the isoelectric point of the amino groups. The charge density should be within the above limits at the pH of intended use. Any anionic counterions can be utilized for the cationic polymers so long as the water solubility criteria is met. Suitable counterions include halides (e. g., Cl, Br, I, or F, preferably Cl, Br, or I), sulfate, and methylsulfate. Others can also be used, as this list is not exclusive. The cationic nitrogen-containing moiety will be present generally as a substituent, on a fraction of the total monomer units of the cationic hair conditioning polymers. Thus, the cationic polymer can comprise copolymers, terpolymers etc. of quaternary ammonium or cationic amine-substituted monomer units and other non-cationic units referred to herein as spacer monomer units.

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. The alkyl and dialkyl substituted monomers preferably have C1-C7 alkyl groups, more preferably C1-C3 alkyl groups.

Other suitable spacer monomers include vinyl esters, vinyl, alcohol (made by hydrolysis of polyvinyl acetate), maleic anhydride, propylene glycol, and ethylene glycol.

The cationic amines can be primary, secondary, or tertiary amines, depending upon the particular species and the pH of the composition. In general, secondary and tertiary amines, especially tertiary, amines, are preferred. Amine-substituted vinyl monomers can be polymerized in the amine form, and then optionally can be converted to ammonium by a quaternization reaction. Amines can also be similarly quaternized subsequent to formation of the polymer. For example, tertiary amine functionalities can be quaternized by reaction with a salt of the formula R'X wherein R' is a short chain alkyl, preferably a C1-C7 alkyl, more preferably a C1-C3 alkyl, and X is an anion which forms a water soluble salt with the quaternized ammonium.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e. g. alkyl vinyl imidazolium, alkyl vinyl pyridinium, alkyl vinyl pyrrolidone salts. The alkyl portions of these monomers are preferably lower alkyls such as the C1-C3 alkyls, more preferably C1 and C2 alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C1-C7 hydrocarbyls, more preferably C1-C3 alkyls.

The cationic polymers hereof can comprise mixtures of monomer units derived from amine- and/or quaternary ammonium-substituted monomer and/or compatible spacer monomers. Suitable cationic hair conditioning polymers include, for example: copolymers of 1-vinyl-2-pyrrolidone and 1-vinyl-3-methylimidazolium salt (e. g. chloride salt) (referred to in the industry by the Cosmetic, Toiletry, and Fragrance Association, "CTFA", as POLYQUATERNIUM-16), such as those commercially available from BASF Wyandotte Corp. (Parsippany, NJ, USA) under the LUVIQUAT tradename (e. g. LUVIQUAT FC 370); co-polymers of 1-vinyl-2-pyrrolidone and dimethylaminoethyl methacrylate (referred to in the industry by CTFA as POLYQUATERNIUM-11) such as those commercially available from Gaf Corporation (Wayne, NJ, USA) under the GAFQUAT tradename (e.g., GAFQUAT 755N); cationic diallyl quaternary ammonium-containing polymers, including, for example, dimethyl diallyl ammonium chloride homopolymer and copolymers of acrylamide and dimethyl diallyl ammonium chloride, referred to in the industry (CTFA) as POLYQUATERNIUM-6 and POLYQUATERNIUM-7, respectively; and mineral acid salts of amino-alkyl esters of homo- and co-polymers of unsaturated carboxylic acids having from 3 to 5 carbon atoms, as described in U.S. Patent 4,009,256, incorporated herein by reference. Other cationic polymers that can be used include polysaccharide polymers, such as cationic cellulose derivatives and cationic starch derivatives. Cationic polysaccharide polymer materials suitable for use herein include those of the formula (II): 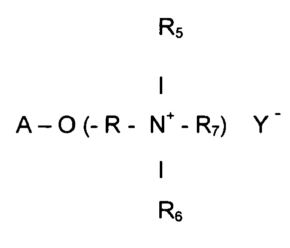 wherein: A is an anhydroglucose residual group, such as a starch or cellulose anhydroglucose residual, R is an alkyene, oxyalkylene, polyoxyalkylene or hydroxyalkylene group, or combination thereof, R₅, R₆, and R₇ independently are alkyl, aryl, alkylaryl, arylalkyl, alkoxyalkyl, or alkoxyaryl groups, each group containing 1 to about 18 carbon atoms, and the total number of carbon atoms for each cationic moiety (i. e. the sum of carbon atoms in R₅, R₆ and R₇) preferably being about 20 or less, and Y is an anionic counterion, as previously described.

Cationic cellulose is available from Amerchol Corp. (Edison, NJ, USA) in their Polymer JR^{®} and LR^{®} series of polymers, as salts of hydroxyethyl cellulose reacted with trimethyl ammonium substituted epoxide, referred to in the industry (CTFA) as POLYQUATERNIUM-10.

Another type of cationic cellulose includes the polymeric quaternary ammonium salts of hydroxyethyl cellulose reacted with lauryl dimethyl ammonium-substituted epoxide, referred to in the industry (CTFA) as POLYQUATERNIUM-24. These materials are available from Amerchol Corp. (Edison, NJ, USA) under the tradename Polymer LM-200.

Other cationic polymers that can be used include cationic quar gum derivatives, such as CTFA: GUAR HYDROXYPROPYL TRIMONIUM CHLORIDE (commercially available from Celanese Corp. in their JaguarR^{®} series).

Other materials include quaternary nitrogen-containing cellulose ethers (e. g. as described in U.S. Patent 3,962,418, incorporated by reference herein), and copolymers of etherified cellulose and starch (e. g. as described in U.S. Patent 3,958,581, incorporated by reference herein).

As discussed above, the cationic polymer hereof is water soluble. This does not mean, however, that it must be soluble in the composition. Preferably however, the cationic polymer is either soluble in the composition, or in a complex coacervate phase in the composition formed by the cationic polymer and anionic material. Complex coacervates of the cationic polymer can be formed with anionic surfactants or with anionic polymers that can optionally be added to the compositions hereof (e. g. sodium polystyrene sulfonate).

The preferred cationic polymers are CTFA: QUATERNIUM-10 and GUAR HYDROXYPROPYL TRIMONIUM CHLORIDE.

The thickener can preferably be used in the emulsion as well as in the hair treating composition of the invention at levels of from about 0.1% to about 10% by weight of the composition, preferably from about 0.3% to about 5%, more preferably from about 0.3% to about 3%, most preferably from about 0.5% to about 1.5 % by weight.

The weight ratio of said crosslinked organopolysiloxane elastomer to said thickener is preferably from about 1 : 1 to about 3 : 1, more preferably from about 2 : 1 to about 2.5 : 1 and most preferred from about 1.5 : 1 to about 2:1, whereby the optimum is about 1.8.

### Water

The emulsion of the invention preferably contains about 70% by weight to about 98% by weight of the emulsion of water, more preferably from about 80 to about 95% by weight, and most preferably from about 85 to about 95% by weight, of the total weight of the emulsion of water.

### Other solvents

The emulsion as well as the hair treating composition may optionally include other liquid, water-miscible or water-soluble solvents such as phenoxyethanol, lower alkyl alcohols, e. g. C1-C5 alkyl monohydric alcohols, preferably C2-C3 alkyl alcohols, most preferred ethanol or isopropanol.

The water-soluble polyhydric alcohols usable in the present invention are also polyhydric alcohols having two or more hydroxyl groups in the molecule. Typical examples of such polyhydric alcohols are dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; penta-hydric alcohols such as xylytol, etc.; hexahydric alcohols such as sorbitol, mannitol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; glycerin monoalkyl ethers such as xyl alcohol, selachyl alcohol, batyl alcohol; sugar alcohols such as sorbitol, maltitol, maltotriose, mannitol, sucrose, erythritol, glucose, fructose, starch sugar, maltose, xylytose, starch sugar reduced alcohol, glysolid, tetrahydrofurfuryl alcohol, POE tetrahydrofurfuryl alcohol, POP butyl ether, POP POE butyl ether, tripolyoxypropylene glycerin ether, POP glycerin ether, POP glycerin ether phosphoric acid, POP POE pentanerythritol ether.

The hair treating composition optionally preferably contains about 0.5 to about 12% by weight, more preferably from about 0.8 to about 10% by weight, and most preferably from about 2.0 to about 8% by weight of the other solvent.

The hair conditioning agent is selected from the group of cationic surfactants, cationic or nonionic silicone compounds and other conditioning agents or mixtures thereof as specified below:

### Cationic Surfactant Conditioning Agents

Cationic surfactants that can be preferably used in the cosmetic composition of the present invention contain amino or quaternary ammonium moieties. Among the quaternary ammonium-containing cationic surfactant materials useful herein are those of the general formula (I)

[NR1,R2,R3,R4]⁺ · X⁻

wherein R1 to R4 are independently an aliphatic group of from about 1 to about 22 carbon atoms or an aromatic, alkoxy, polyoxyalkylene, alkylamido, hydroxyalkyl, aryl or alkylaryl group having from about 1 to about 22 carbon atoms; and X⁻ is a salt-forming anion such as those selected from halogen, (e. g. chloride, bromide, iodide), acetate, citrate, lactate, glycolate, phosphate nitrate, sulfate, and alkylsulfate radicals.

The aliphatic groups may contain, in addition to carbon and hydrogen atoms, either linkages, and other groups such as amino groups. The longer chain aliphatic groups, e. g. those of about 12 carbons, or higher, can be saturated or unsaturated. Especially preferred are di-long chain (e.g., di C12-C22, preferably C16-C18, aliphatic, preferably alkyl), di-short chain (e.g. C1 - C3 alkyl, preferably C1 - C2 alkyl) ammonium salts. Salts of primary, secondary and tertiary fatty amines are also suitable cationic surfactant materials. The alkyl groups of such amines preferably have from about 12 to about 22 carbon atoms, and may be substituted or unsubstituted. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydorxy ethyl stearylamine, and arachidylbehenylamine. Suitable amine salts include the halogen, acetate, phosphate, nitrate, citrate, lactate, and alkyl sulfate salts. Such salts include stearylamine hydrochloride, soyamine chloride, stearylamine formate, N-tallowpropane diamine dichloride and stearamidopropyl dimethylamine citrate. Preferred cationic surfactants are cetyl trimethyl ammonium salts, as for example Genamin^{®} CTAC, i. e. cetyl trimethyl ammonium chloride, behenyl trimethyl ammonium salts, e. g. behenyl trimethyl ammonium chloride; dimethyl ditallow ammonium salts; stearyl amidopropyl dimethylamine; esterquats as for example tetradecyl betainester chloride, diesterquats as for example dipalmitylethyl dimethylammoniumchloride (Armocare^{®} VGH70 of Akzo, Germany), or a mixture of distearoylethyl hydroxyethylmonium methosulfate and Cetearyl Alkohol (Dehyquart^{®} F-75 of Henkel, Germany).

Cationic surfactants are preferably contained at levels of from about 0.1 % to about 5%, more preferably from about 0.2% to about 1,5%, most preferably from about 0.4% to about 0.8%, by weight of the composition. These cationic surfactants however preferably should not be present in the composition of the invention if no fatty alcohol is present, but may be contained also in this case up to a level up to about 0.1 % by weight without disadvantage.

### Silicone Conditioning Agents

The cosmetic compositions hereof can also include volatile or nonvolatile, soluble or insoluble silicones as conditioning agents. By soluble what is meant is that the silicone conditioning agent is miscible with the aqueous carrier of the composition so as to form part of the same phase. By insoluble what is meant is that the silicone from a separate, discontinuous phase from the aqueous carrier, such as in the form of an emulsion or a suspension of droplets of the silicone.

Soluble silicones include silicone copolyols, such as dimethicone copolyols, e.g. polyether siloxane-modified polymers, such as polypropylene oxide, polyethylene oxide modified polydimethylsiloxane, wherein the level of ethylene and/or propylene oxide sufficient to allow solubility in the composition.

Preferred, however, are insoluble silicones. The insoluble silicone hair conditioning agent for use herein will preferably have viscosity of from about 1,000 to about 2,000,000 mPa · s at 25°C, more preferably from about 10,000 to about 1,800,000, even more preferably from about 100,000 to about 1,500,000 mPa · s at 25°C. The viscosity can be measured by means of a glass capillary viscometer as set forth in Dow Corning Corporate Test Method CTM0004, July 20, 1970.

Suitable insoluble, nonvolatile silicone fluids include polyalkyl siloxanes, polyaryl siloxanes, polyalkylaryl siloxanes, polyether siloxane copolymers, dimethylpolysiloxane containing terminal hydroxyl groups, methylphenyl polysiloxane containing terminal hydroxyl groups and mixtures thereof. Specific examples thereof include hexamethyldisiloxane, octamethyltrisiloxane, decamethyltetrasiloxane and hexadecamethylheptasiloxane.

Other insoluble, nonvolatile silicone fluids having hair conditioning properties can also be used. The term "nonvolatile" as used herein shall mean that the silicone has a boiling point of at least about 260°C, preferably at least about 275°C, more preferably at least about 300°C. Such materials exhibit very low or no significant vapor pressure at ambient conditions. The term "silicone fluid" shall mean flowable silicone materials having a viscosity of less than 1,000,000 mPa · s at 25°C. Generally, the viscosity of the fluid will be between about 5 and 1,000,000 mPa·s at 25°C, preferably between about 10 and about 300,000 mPa · s at 25°C.

The preferred silicones are polydimethyl siloxane, polydiethylsiloxane, and polymethylphenylsiloxane. Polydimethylsiloxane is especially preferred. The nonvolatile polyalkylsiloxane fluids that may be used include, for example, polydimethyl siloxanes. These siloxanes are available, for example, from the General Electric Company in their ViscasilR and SF 96 series, and from Dow Corning in their Dow Corning 200^{®} series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid or diquaternary silikones as for example CTFA: QUATERNIUM-80 (e. g. Abil^{®} Quat 3272 or Abil^{®} Quat 3270 of Th. Goldschmidt AG, Germany).

Suitable insoluble, volatile silicone fluids include low molecular weight oligomeric polydimethylsiloxane or cyclic polydimethylsiloxane, having a viscosity of no more than 10 mPa · s at 25°C and a boiling point under atmospheric pressure of no more than 250°C. Volatility can be achieved in linear organopolysiloxanes by selection of oligomeric organopolysiloxanes with at most 6 to 10 silicone atoms in the organopolysiloxanes backbone, e. g. Dow Corning DC200 Fluid, having a viscosity of from about 0.65 to about 2 mPa·s at 25°C. Preferably cylclic organopolysiloxanes having from 3 to 6 silicon atoms are utilized, for example, hexamethylcyclotrisiloxane, octamethylcyclotetrasiloxane, decamethylcyclopentasiloxane, dodecamethylcyclohexasiloxane (e. g. DC 244, DC 245, DC 345 of Dow Corning).

Especially preferred, for enhancing the shine characteristics of hair, are highly arylated silicones, such as highly phenylated polyethyl silicone having refractive indices of about 1.46 or higher, especially about 1.52 or higher. When these high refractive index silicones are used, they should be mixed with a spreading agent, such as a surfactant or a silicone resin, as described below to decrease the surface tension and enhance the film forming ability of the material.

The polyether siloxane copolymers that may be used include, for example, a polypropylene oxide modified polydimethylsiloxane (e. g. Dow Corning DC-1248^{®}) although ethylene oxide or mixtures of ethylene oxide and propylene oxide may also be used. The ethylene oxide and polypropylene oxide level should be sufficiently low to prevent solubility in the composition hereof.

Another silicone hair conditioning material that can be especially useful in the silicone conditioning agents is insoluble silicone gum. The term "silicone gum", as used herein, means polyorganosiloxane materials having a viscosity at 25°C of greater than or equal to 1,000,000 mPa · s. Silicone gums are described by Petrarch and others including U.S. Patent 4,152,416. These described references are incorporated herein by reference. The "silicone gums" will typically have a mass molecular weight in excess of about 200,000, generally between about 200,000 and about 1,000,000. Specific examples include polydimethylsiloxane, (polydimethylsiloxane) (methylvinylsiloxane) copolymer, poly(dimethylsiloxane) (diphenyl siloxane) (methylvinylsiloxane) copolymer and mixtures thereof. Preferably the silicone hair treating agent comprises a mixture of a polydimethylsiloxane gum, having a viscosity greater than about 1,000,000 mPa·s and polydimethylsiloxane fluid having a viscosity of from about 10 mPa · s to about 100,000 mPa · s at 25°C, wherein the ratio of gum to fluid is from about 30:70 to about 70:30, preferably from about 40:60 to about 60:40.

Further silicones for use herein which are preferred are amino functional siloxanes which conform to the general formula (III) 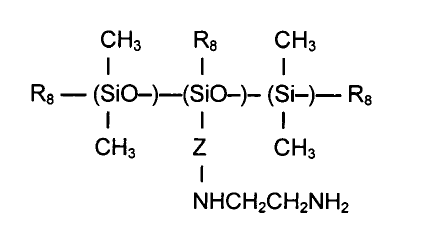 wherein R₈ = OH or CH₃ and Z represents the propyl, isopropyl or isobutyl group. These silicones, e. g., copolymer of aminoethyl aminopropyl siloxane and dimethyl siloxane are available from Dow Corning and sold under the trade name Dow Corning 2-8566 Amino Fluid^{(R)} or as a mixture with polyethylenglycol ether of tridecyl alcohol and cetyl trimethyl ammoniumchloride, sold as Dow Corning 929 Cationic Emulsion^{(R)}.

Silicone polymers that are specially preferred are CTFA: QUATERNIUM-80 and AMO-DIMETHICONE. Also preferred are volatile silicones such as e. g. CTFA: DIMETHICONE and CYCLOMETHICONE.

The silicone hair conditioning agent can be used in the compositions hereof at levels of from about 0.1 % to about 20% by weight of the composition, preferably from about 0.5 % to about 15%, more preferably from about 1% to about 10% and most preferably from about 3% to about 8% by weight.

The high molecular weight silicone according to the invention prevents and repairs damage such as split hair and torn hair by coating the damaged portion of the hair surface with a thin film of a high molecular weight silicone.

### Additional Conditioning Agents

The cosmetic compositions of the present invention can also comprise one or more additional conditioning agents, such as those selected from the group consisting of liquid oils and fats such as avocado oil, tsubaki oil, turtle oil, Macademia nuts oil, corn oil, mink oil, olive oil, rape seed oil, yolk oil, sesame oil, parsic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, hohoba oil, germ oil, triglycerine, trioctanoic acid glycerine, triisopalmitic acid glycerine; solid fats such as cacao fat, coconut oil, horse fat, hardened coconut fat, palm oil, tallow, sheep fat, hardened tallow, palm kernel oil, jojoba oil, lard, ox bone fat, wood wax kernel oil, hardened castor oil; waxes such as beeswax, apple wax, canderilla wax, cotton wax, carunauba wax, bayberry wax, insect wax, whale wax, montan wax, rice bran wax, lanolin, kapok wax, lanolin acetate, liquid lanolin, cane wax, isopropyl lanolin fatty acid, hexyllaurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether; hydrocarbons , nonvolatile hydrocarbons and hydrocarbon esters such as fluid paraffin, solid paraffin, vaseline, ozocerite, squalane, pristan, ceresin, squalane, petrolatum, isododecane, microcrystalline wax; fatty acid oils, ester oils such as cetyl octanoate, isopropyl myristate; betaine, carnitin, carnitin esters, creatine, amino acids, peptides, proteines, vitamines, phospholipides, e. g. lecithines or ceramides. Useful are also imidazolidinyl derivatives as for example (CTFA) QUATERNIUM-87 (Rewoquat^{®} W 575 of Witco, Germany). The amount of fatty substances preferably ranges from about 2 to 30% by weight, better still from about 5 to 15% by weight and most preferred from about 7 to 10% by weight of the total weight of the cosmetic composition. These ranges include all specific values and subranges there between, including 3, 8, 10, 15, 20, 25 and 30% by weight.

### Fatty Alcohols

The cosmetic compositions of the present invention may also comprise a nonvolatile low melting point fatty alcohol. The fatty alcohols hereof have a melting point of 30°C or less, preferably about 25°C or less, more preferably about 22°C or less. The unsaturated fatty alcohols hereof are also nonvolatile. By nonvolatile what is meant is they have a boiling point at 1.0 atmospheres of at least about 260°C, preferably at least about 275°C, more preferably at least about 300°C. Suitable fatty alcohols include unsaturated monohydric straight chain fatty alcohols, saturated branched chain fatty alcohols, saturated C8-C12 straight chain fatty alcohols, and mixtures thereof. The unsaturated straight chain fatty alcohols will typically have one degree of unsaturation. Di- and tri- unsaturated alkenyl chains may be present at low levels, preferably less than about 5% by total weight of the unsaturated straight chain fatty alcohol more preferably less than about 2%, most preferably less than about 1%. Preferably, the unsaturated straight chain fatty alcohols will have an aliphatic chain size of from C12-C22, more preferably from C12-C18, most preferably from C16-C18. Exemplary alcohols of this type include oleyl alcohol, and palmitoleic alcohol. The branched chain alcohols will typically have aliphatic chain sizes of from C12-C22, preferably C14-C20, more preferably C16-C18.

Exemplary branched chain alcohols for use herein include stearyl alcohol, cetyl alkohol, isostearyl alcohol, octyl dodecanol, and octyl decanol.

Examples of saturated C8-C12 straight chain alcohols include octyl alcohol, caprylic alcohol, decyl alcohol, and lauryl alcohol. The low melting point fatty alcohols hereof are used at a level of from about 0.1 % to about 10%, by weight of the composition, more preferably from about 0.2% to about 5%, most preferably from about 0.5% to about 3%.

The present cosmetic compositions are preferably limited to levels of monohydric saturated straight chain fatty alcohols, such as cetyl alcohol and stearyl alcohol, and other waxy fatty alcohols having melting points above 45°C, of no more than about 5%, by weight of the composition, preferably no more than about 4% since the presence of such waxy fatty alcohols can adversely affect the shine benefits of the present invention.

However, it may be desirable to use waxy fatty alcohols for their conditioning benefits. In the event that such saturated fatty alcohols are present, the weight ratio of the liquid to waxy fatty alcohols is preferably no greater than about 0.25, more preferably no greater than about 0.15, more preferably than about 0.10.

The total amount of fatty alcohols in the composition is preferably about 0.5 to about 5.0 % by weight, more preferably from about 1.0 to about 4.0% by weight, and most preferably from about 1.5 to about 3.0% by weight.

### Other Ingredients

The cosmetic compositions herein can contain a variety of other optional components suitable for rendering such compositions more cosmetically or aesthetically acceptable or to provide them with additional usage benefits. Such conventional optional ingredients are well-known to those skilled in the art. The composition of the invention may thus comprise lipophilic or hydrophilic adjuvants which are standard in the cosmetics or dermatological fields, such as surfactants, in particular foaming surfactants, preservatives, antioxidants, sequestering agents, solvents, fragrances, fillers, screening agents, odor absorbers, coloring materials and lipid vesicles.

A wide variety of additonal ingredients can be formulated into the present cosmetic composition. These include: hair-hold polymers, detersive surfactants such as anionic, nonionic, amphoteric, and zwitterionic surfactants; additional thickening agents and suspending agents, such as xanthan gum, guar gum, starch and starch derivatives, viscosity modifiers such as methanolamides of long chain fatty acids, cocomonoethanol amide, salts such as sodium potassium chloride and sulfate and crystalline suspending agents, and pearlescent aids such as ethylene glycol distearate; UV-filters and sunscreens, e. g. such as p-methoxy cinnamic acid isoamylester, lipophilc cinnamic acid esters, salicylic acid esters, 4-amino benzoic acid derivatives or hydrophilic sulfonic acid derivatives of benzophenones or 3-benzyliden campher; antioxidants such as tocopheroles; agents for combating free radicals; preservatives such as benzyl alcohol, methyl paraben, propyl paraben and imidazolidinyl urea; polyvinyl alcohol; pH adjusting agents, such as citric acid, formic acid, glyoxylic acid, acetic acid, lactic acid, pyruvic acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents, such as the thioglycolates; perfumes, sequestering agents, such as disodium ethylenediamine tetra-acetate, and polymer plasticizing agents, such as glycerin, disobutyl adipate, butyl stearate, and propylene glycol.

From the nonionic surfactants optionally used those are prferred having an HLB (Hydrophilic Lipophilic Balance) of greater than 12 and the primary emulsion comprises at least one nonionic surfactant having an HLB of less than 8. The nonionic surfactant of HLB>12 optionally present in the emulsion may be, for example, an ethoxylated or ethoxylated/propoxylated fatty alcohol with a fatty chain comprising from 12 to 22 carbon atoms, ethoxylated sterols, such as stearyl- or lauryl alcohol (EO-7); PEG-16 soya sterol or PEG-10 soya sterol, polyoxyethylene polyoxypropylene block polymers (poloxamers) and their mixtures. Ethoxylated sterols and of poloxamers are preferred. The nonionic surfactant of HLB<8 can be chosen in particular from glyceryl esters, such as mono-, di- or triglyceryl mono-, di- or triisostearate or -oleate, sugar esters, such as sucrose or methyl glucose mono- or diisostearate or -oleate, alkylpolyglucoside ethers, such as sorbitan isostearate, oleyl- or isostearylpolyglucoside; polyoxyethylene (20) sorbitan monostearate (CTFA: Polysorbat-60), and their mixtures. Sugar esters and alkylpolyglucoside ethers are preferred.

The amount of nonionic surfactant in the emulsion and the cosmetic composition preferably can range from about 0.1 to about 5% by weight and more preferably from about 1 to about 3% by weight of the total weight of the emulsion.

The emulsion and the cosmetic composition of the invention preferably does not comprise any anionic or amphoteric surfactant. Such an anionic or amphoteric surfactant may be present in the emulsion however in an amount up to about 0.2% by weight of the cosmetic composition without disadvantage.

Other optional ingredients and those cited above generally are used individually at levels from about 0.01% to about 10.0%, preferably from about 0.05% to about 5.0% of the cosmetic composition.

These adjuvants, depending on their nature, can be introduced into the oily phase or into one of the aqueous phases.

### VISCOSITY

The hair treating composition of the present invention preferably has a viscosity at 25°C of at least about 10 mPa · s, preferably from about 50 mPas to about 10,000 mPas, more preferably from about 500 mPa·s to about 6,000 mPa · s and even more preferably from about 1,500 mPa·s to 4,500 mPa·s. Viscosity is determined - if not otherwise defined - by HAAKE Rotation Viscometer VT 550 with cooling/heating vessel and sensor systems according to DIN 53019 (MV-DIN, SV-DIN), shear rate is 12.9 s⁻¹.

The cosmetic composition according to the invention may also be in the form of, for example, protective, treatment or care creams for the face, for the hands or for the feet, protective or care body milks, or lotions, gels or foams for caring for the skin, mucous membranes, hair and scalp.

### METHOD OF USE

The emulsions of the invention can be used in various topical applications, in particular cosmetic and/or dermatological applications. The composition based on this emulsion can constitute in particular compositions for cleansing, protecting, treating and/or caring for the skin, mucous membranes and/or hair, in particular for the face, for the neck, for the hands, for the hair, for the scalp or for the body, as well as for the eyelashes. An effective amount of the emulsion or a composition containing the emulsion may be applied to skin, mucous membranes and/or hair desired to be treated.

The inventive emulsion may be a further subject-matter of the invention is consequently the cosmetic use of the composition according to the invention for cleansing and/or treating and/or protecting the skin and/or mucous membranes and/or keratinous fibers, that is to say the hair and/or eyelashes.

Included in the present invention is the use of the composition according to the invention for the preparation of a composition intended to cleanse and/or treat and/or protect the skin and/or mucous membranes and/or keratinous fibers, that is to say the hair and/or eyelashes.

Another facet of the present invention is a process for cleansing and/or treating and/or protecting the skin, mucous membranes and/or keratinous fibers, characterized in that it consists in applying a composition as defined above to the skin, mucous membranes and/or keratinous fibers.

The hair care compositions of the present invention are used in conventional ways to provide the conditioning and shine benefits of the present invention. Such method of use depends upon the type of composition employed but generally involves application of an effective amount of the product to the hair, which may then be rinsed from the hair (as in the case of hair rinses) or allowed to remain on the hair (as in the case of gels, lotions, and creams). By "effective amount" is meant an amount sufficient enough to provide a hair shine benefit. In general, from about 1g to about 50g is applied to the hair on the scalp. The composition is distributed throughout the hair typically by rubbing or massaging the hair and scalp with ones' hands or by another's hands. Preferably, the composition is applied to wet or damp hair prior to drying of the hair. After such compositions are applied to the hair, the hair is dried and styled in accordance with the desires of the user and in the usual ways of the user. Alternately, the composition is applied to dry hair, and the hair is then combed or styled in accordance with the desires of the user.

### PROCESS OF MANUFACTURING OF THE EMULSION

The O/W and/or W/O/W multiple emulsion, or the mixture thereof, may be prepared according to two methods:

### Method 1 : Stirring

(a) Mixing the oily components, including the organopolysiloxane crosslinked with polyether chains, by stirring until the oil phase was homogeneous,
(b) preparing the aqeous phase including the thickener by stirring the mixture until the gel becomes clear and
(c) adding the water-phase slowly to the oil phase by stirring the mixture until the mass is homogeneous.

### Method 2 : Homogenizing

### (a) Provide all components in a container and (b) homogenize the mixture.

The homoginization can preferably be carried out at a teperature of from about 20 to about 90°C, more preferably from about 25 to about 80°C, using a preferred stirring rate of from about 4,000 to about 20,000 rpm, more preferably from about 6,000 to about 14,000 rpm. The duration of the homoginization may be preferably from about 1 to about 6 minutes, more preferably from about 2 to about 4 minutes.

In the most preferred method for homogenization a Rotor-Stator-Homogenizer (e. g. Ultra Turrax) is used; for other suitable methods a High Pressure Gap Homogenizer or a Ultrasonic Homogenizer is used. The homogenizing temperature depends on the raw-materials. The time and the number of homogenization cycles are also depending on the raw materials, at a cold/cold process, one cycle is preferred with about 4,000 to about 20,000 rpm, more preferred from about 6,000 to about 14,000 rpm. The applicable temperature range for the cold/cold process is from about 20°C to about 40°C. In the case of a hot/hot process, which is also applicable, the temperature ranges from about 60 to about 90°C, preferably with from about 70 to about 80°C. During the hot/hot process, two cycles are preferred, one at about 60 to 75°C for about 3 to about 6 minutes and a second one at about 20 to about 40°C for about 2 to about 4 minutes (rpm are the same as for a cold/cold process).

### Method 2 (homogenizing) is preferred.

The homogenization method used for the emulsions in table 2 and all examples, except example 2, was a cold/cold (cold oilphase/ cold waterphase) process, because all raw materials were liquid or soluble at room temperature. Therefore the emulsification took place at a temperature of 25°C. All components were metered in a container and homogenized for 2 minutes with a Rotor-Stator homogenizer (e. g. Ultra Turrax) at 10,000 rpm.

In example 2 the cetyltrimethylammonium chloride was solved in hot water (80°C) and the cetylstearyl alcohol was melted at 80°C. The hot waterphase was metered to the hot wax phase and stirred for 5 minutes at 100 rpm. After cooling down to 40°C all other ingredients were metered into the container and homogenized at that temperature for 2 minutes at 10,000 rpm.

The resulting emulsions are given in the following Table 2 and the examples:

### EXAMPLES

The following examples illustrate the present invention. It will be appreciated that other modifications of the present invention within the skill of those in the hair care formulation art can be undertaken without departing from the spirit and scope of this invention.

All parts, percentages, and ratios herein are by weight unless otherwise specified. Some components may come from suppliers as dilute solutions. The levels given reflect the weight percent of the active material, unless otherwise specified.

### Examples for Preparing Emulsions

| | |
|---|---|
| 5.00 g | Dimethicone |
| 3.00 g | KSG-Type from Table 1 |
| 0.40 g | Polyacrylamide |
| 0.20 g | C13-C14 isoparaffine |
| 0.20 g | lauryl alcohol (EO-7) |
| 0.20 g | PHB-methylester |
| 0.40 g | phenoxy ethanol |
| ad 100.00 g | water |

**Table 1: KSG-Emulsifiers**

| **KSG-Type** | **INCI Name** |
|---|---|
| KSG-210 | 76.00% Dimethicone |
| | 24.00% Dimethicone PEG-10/15 Crosspolymer |
| KSG-310 | 69.48% Mineral Oil |
| | 30.00% PEG-15/Lauryl Dimethicone Crosspolymer |
| | 0.50% Dipropylene Glycol |
| | 0.02% Tocopherol |
| KSG-320 | 74.48% Isododecane |
| | 25.00% PEG-15/Lauryl Dimethicone Crosspolymer |
| | 0.50% Dipropylene Glycol |
| | 0.02% Tocopherol |
| KSG-330 | 79.48%Triethylhexanoin |
| | 20.00% PEG-15/Lauryl Dimethicone Crosspolymer |
| | 0.50% Dipropylene Glycol |
| | 0.02% Tocopherol |
| KSG-340 | 69.48% Squalane |
| | 20.00% PEG-15/Lauryl Dimethicone Crosspolymer |
| | 10.00% PEG-10/Lauryl Dimethicone Crosspolymer |
| | 0.50% Dipropylene Glycol |
| | 0.02% Tocopherol |
| KSG-710 | 74.48% Dimethicone |
| | 25.00% Dimethicone/Polyglycerin-3 Crosspolymer |
| | 0.50% Dipropylene Glycol |
| | 0.02% Tocopherol |
| KSG-810 | 69.48% Mineral Oil |
| | 30.00% Lauryl Dimethicone/ Polyglycerin-3 Crosspolymer |
| | 0.50% Dipropylene Glycol |
| | 0.02% Tocopherol |
| KSG-820 | 74.48% Isododecane |
| | 25.00% Lauryl Dimethicone/ Polyglycerin-3 Crosspolymer |
| | 0.50% Dipropylene Glycol |
| | 0.02% Tocopherol |
| KSG-830 | 80.00% Triethylhexanoin |
| | 19.48% Lauryl Dimethicone/ Polyglycerin-3 Crosspolymer |
| | 0.50% Dipropylene Glycol |
| | 0.02% Tocopherol |
| KSG-840 | 70.00% Squalane |
| | 29.48% Lauryl Dimethicone/ Polyglycerin-3 Crosspolymer |
| | 0.50% Dipropylene Glycol |
| | 0.02% Tocopherol |

According to Method 1 the oil phase with the silicone crosspolymer (KSG-Type), Dimethicone and phenoxy ethanol was prepared by stirring until the mass was homogeneous. The stirring was done at 25°C with 500 rmp. The stirring time was 6 minutes. Than the water phase with water, polyacrylamide, C13-C14 isoparaffine, lauryl alcohol (EO-7) and PHB-methylester was provided and stirred until the gel was homogenous; The stirring was done at 25°C with 500 rmp. The stirring time was 10 minutes. Than the water-phase was added slowly to the oil phase by stirring the mixture until the mass was homogeneous.

The resulting emulsions are given in the following Table 2:

**Table 2 Overview of phasing results**

| **KSG-Typ** | **Manufacturing** | **Phasing** | **Summary** |
|---|---|---|---|
| 210 | Stirred | 80-90% W/O/W 10-20% O/W | With KSG-210 multiple emulsion with a rate of 80-90% W/O/W droplets can be produced. The multiple emulsion evolved independently of the manufacturing procedure and the choice of raw materials. |
| | Homogenized | >90%W/O/W <10%O/W | |
| 310 | Stirred | 80-90%O/W 10-20% W/O/W | Only 10-20% multiple droplets were formed by KSG-310. |
| | Homogenized | 80-90%O/W 10-20% W/O/W | |
| 320 | Stirred | 80-90%O/W 10-20% W/O/W | Only 10-20% multiple droplets were formed by KSG-320. The W/O/W droplets were bigger than the O/W droplets and showed coalescence. |
| | Homogenized | 80-90%O/W 10-20% W/O/W | |
| 330 | Stirred | 90-95%O/W 5-10%W/O/W | Only 5-20% multiple droplets were formed by KSG-330. The droplet size was inhomogeneous. |
| | Homogenized | 80-90%O/W 10-20% W/O/W | |
| 340 | Stirred | 95%O/W 5%W/O/W | With KSG-340 only 5-10% W/O/W droplets were formed. |
| | Homogenized | 90-95%O/W 5-10%W/O/W | |
| 710 | Stirred | 100%O/W | With KSG-710 normal emulsions, O/W.type, without multiple droplets were formed |
| | Homogenized | 100%O/W | |
| 810 | Stirred | 95%O/W 5%W/O/W | With KSG-810 only 5% W/O/W droplets were formed. |
| | Homogenized | 95%O/W 5%W/O/W | |
| 820 | Stirred | 80-90%O/W 10-20% W/O/W | 10-20% of multiple droplets can be generated by KSG-820. The droplets had a corrugated shape. |
| | Homogenized | 90%O/W 10%W/O/W | |
| 830 | Stirred | 95%O/W <5%W/O/W | Only 5-10% of multiple emulsion can be generated by KSG-830. |
| | Homogenized | 90%O/W 10%W/O/W | |
| 840 | Stirred | 95%O/W 5%W/O/W | Only small rates of 5% W/O/W droplets were formed by KSG-840. |
| | Homogenized | 95%O/W 5%W/O/W | |

### Examples for Cosmetic Compositions

### Example 1 Hair Leave-in Treatment

| | |
|---|---|
| 7.50 g | Dimethicone |
| 0.70 g | Dimethicone/PEG-10/15 Crosspolymer (KSG210) |
| 0.40 g | Polyacrylamide |
| 0.20 g | C13-C14 isoparaffine |
| 0.20 g | lauryl alcohol (EO-7) |
| 0.20 g | PHB-methylester |
| 0.40 g | phenoxy ethanol |
| 0.30 g | perfume oil |
| ad 100.00 g | water |
| pH =5.92 | viscosity = 2,514 mPa · s at 25°C |

### Example 2 Hair Conditioning Cream

| | |
|---|---|
| 7.50 g | Dimethicone |
| 0.70 g | Dimethicone/PEG-10/15 Crosspolymer (KSG210) |
| 0.70 g | cetyltrimethylammonium chloride |
| 4.50 g | cetylstearyl alcohol |
| 0.40 g | phenoxy ethanol |
| 0.40 g | isopropyl alcohol |
| 0.20 g | PHB-methylester |
| 0.20 g | perfume oil |
| ad 100.00 g | water |
| pH = 4.86 | viscosity = 2,937 mPa · s at 25°C |

### Example 3 Leave in Conditioner

| | |
|---|---|
| 7.50 g | Dimethicone |
| 0.70 g | Dimethicone/PEG-10/15 Crosspolymer (KSG210) |
| 0.60 g | Hydroxyethyl acrylate/sodium Acryloyldimethyl taurate copolymer (Simulgel^{®} NS) |
| 0.40 g | squalane |
| 0.20 g | C13-C14 isoparaffine |
| 0.05 g | lauryl alcohol (EO-7) |
| 0.20 g | PHB-methylester |
| 0.40 g | phenoxy ethanol |
| 0.08 g | polyoxyethylene (20) sorbitan monostearate (CTFA: Polysorbat- |
| | 60) |
| 0.02 g | sorbitan isostearate |
| 0.30 g | perfume oil |
| ad 100.00 g | water |
| pH = 5.47 | viscosity = 3,175 mPa · s at 25°C |

### Example 4 Watercream for Haircare

| | |
|---|---|
| 3.00 g | Dimethicone |
| 0.40 g | Dimethicone Crosspolymer (KSG330) |
| 0.40 g | Polyacrylamide |
| 0.20 g | C13-C14 isoparaffine |
| 0.05 g | lauryl alcohol (EO-7) |
| 0.20 g | PHB-methylester |
| 0.40 g | phenoxy ethanol |
| 2.00 g | isopropyl myristate |
| 1.60 g | glyceryl tri (2-ethylhexanoate) (CTFA: TRIETHYLHEXANOIN) |
| 0.50 g | perfume oil |
| ad 100.00 g | water |
| pH = 5.87 | viscosity = 1,857 mPa · s at 25°C |

### Example 5 Humectant Emulsion

| | |
|---|---|
| 2.30 g | Dimethicone |
| 0.70 g | Dimethicone/PEG-10/15 Crosspolymer (KSG210) |
| 0.40 g | Polyacrylamide |
| 0.20 g | C13-C14 isoparaffine |
| 0.05 g | lauryl alcohol (EO-7) |
| 5.00 g | butylene glycol |
| 0.20 g | PHB-methylester |
| 0.40 g | phenoxy ethanol |
| 0.30 g | perfume oil |
| ad 100.00 g | water |
| pH = 4.79 | viscosity = 3,813 mPa · s at 25°C |

### Example 6 Humectant Emulsion

| | |
|---|---|
| 2.30 g | Dimethicone |
| 0.70 g | Dimethicone/PEG-10/15 Crosspolymer (KSG210) |
| 0.40 g | Polyacrylamide |
| 0.20 g | C13-C14 isoparaffine |
| 0.05 g | lauryl alcohol (EO-7) |
| 0.20 g | PHB-methylester |
| 10.00 g | propylene glycol |
| 0.40 g | phenoxy ethanol |
| 0.30 g | perfume oil |
| ad 100.00 g | water |
| pH = 5.07 | viscosity = 2,764 mPa · s at 25°C |

### Example 7 Watercreme for Hair

| | |
|---|---|
| 5.00 g | Dimethicone |
| 0.40 g | Dimethicone Crosspolymer (KSG330) |
| 0.40 g | Polyacrylamide |
| 0.20 g | C13-C14 isoparaffine |
| 0.05 g | lauryl alcohol (EO-7) |
| 0.20 g | PHB-methylester |
| 0.01 g | polyetylene glycol (CTFA: PEG-90M) |
| 1.60 g | glyceryl tri (2-ethylhexanoate) |
| 0.40 g | phenoxy ethanol |
| 0.30 g | perfume oil |
| ad 100.00 g | water |
| pH = 6.43 | viscosity = 1,866 mPa · s at 25°C |

### Example 8 Leave-in Hair Conditioning Treatment

| | |
|---|---|
| 5.00 g | Dimethicone |
| 0.40 g | Dimethicone Crosspolymer (KSG330) |
| 0.40 g | Polyacrylamide |
| 0.20 g | C13-C14 isoparaffine |
| 0.05 g | lauryl alcohol (EO-7) |
| 0.20 g | PHB-methylester |
| 1.00 g | polyvinyl pyrrolidone |
| 1.60 g | glyceryl tri (2-ethylhexanoate) |
| 0.40 g | phenoxy ethanol |
| 0.50 g | perfume oil |
| ad 100.00 g | water |
| pH = 4.44 | viscosity = 4,151mPa · s at 25°C |

### Example 9 Watercream Hair Conditioning Treatment

| | |
|---|---|
| 2.30 g | Dimethicone |
| 0.70 g | Dimethicone/PEG-10/15 Crosspolymer (KSG210) |
| 0.40 g | Polyacrylamide |
| 0.20 g | C13-C14 isoparaffine |
| 0.05 g | lauryl alcohol (EO-7) |
| 0.20 g | PHB-methylester |
| 5.00 g | isododecane |
| 0.40 g | phenoxy ethanol |
| 0.30 g | perfume oil |
| ad 100.00 g | water |
| pH = 5.21 | viscosity = 2,833 mPa · s at 25°C |

## Claims

1. An oil-in-water and/or water/oil/water multiple emulsion, comprising
(A) a organopolysiloxane elastomer crosslinked with polyether chains and
(B) at least one thickener selected from the group of
(a) a polyacrylamide homo- or copolymer,
(b) a celluloseether,
(c) a non-silicone cationic polymer or
(d) a thickening system comprising a mixture of from about 60 to about 67% of the thickening system of a copolymer of 2-propenoic acid with 2-propenamide, from about 27 to about 32% of the thickening system of the homopolymer of 2-methyl-1-propene and from about 5 to about 7% of the thickening system of poly(oxy-1,2-ethanediyl)-sorbitan-monododecanoate.

2. The emulsion of claim 1, **characterized in that** said polyether chain is a polyglycerine chain.

3. The emulsion according to claim 1 or 2, **characterized in that** said crosslinked organopolysiloxane elastomer displays at least one alkyl chain having 1 to 20 carbon atoms.

4. The emulsion according to claim 3, **characterized in that** said alkyl chain has 2 to 6 carbon atoms.

5. The emulsion according to any one of claims 1 to 4, **characterized in that** said thickener is a polyacrylamide homopolymer.

6. The emulsion according to any one of claims 1 to 5, **characterized in that** it comprises at least one oil selected from animal oils, vegetable oils, mineral oils, synthetic oils, waxes.

7. The emulsion according to any one of claims 1 to 6, wherein the emulsion comprises from about 0.5 % by weight to about 10% by weight of said crosslinked organopolysiloxane elastomer.

8. The emulsion according to any one of claims 1 to 7, wherein the emulsion comprises from about 0.5 % by weight to about 10% by weight of said crosslinked organopolysiloxane elastomer.

9. The emulsion according to any one of claims 1 to 8, wherein the emulsion comprises from about 1 % by weight to about 5% by weight of said crosslinked organopolysiloxane elastomer.

10. The emulsion according to any one of claims 1 to 9, wherein the emulsion comprises from about 0.1 % by weight to about 3% by weight of said thickener.

11. The emulsion according to any one of claims 1 to 10, wherein the emulsion comprises from about 70 % by weight to about 98% by weight of water.

12. The emulsion according to any one of claims 1 to 11, wherein the emulsion comprises from about 80 % by weight to about 95% by weight of water.

13. Cosmetic composition to be applied to the skin or to the hair, comprising an emulsion as defined in any one of claims 1 to 12.

14. A hair conditioning composition comprising an emulsion as defined in any one of claims 1 to 13 and a hair conditioning agent.

15. Composition as in claim 14, comprising as hair conditioning agent a cationic surfactant.

16. Composition as in claim 15, wherein said cationic surfactant, is selected from the group consisting of cetyl trimethyl ammonium salts, behenyl trimethyl ammonium salts, dimethyl ditallow ammonium salts and stearyl amidopropyl dimethylamine.

17. The composition of claim 13 comprising an active agent.

18. The composition of claim 17, wherein the active agent is selected from the group consisting of polyols, vitamins, screening agents, moisturizers, enzymes, natural extracts, procyanidol oligomers, carotenoids, polyunsaturated fatty acids, α-hydroxy acids, β-hydroxy acids, plant extracts comprising such an acid, and mixtures thereof.

19. The composition of claim 18, wherein the active agent is selected from the group consisting of retinoic acid, vitamin C, vitamin A (retinol), urea, rutin, ascorbic acid, salicylic acid, lactic acid, methyllactic acid, citric acid, kojic acid, caffeic acid, mandelic acid, glucuronic acid, glycolic acid, pyruvic acid, 2-hydroxybutanoic acid, 2-hydroxypentanoic acid, 2-hydroxyhexanoic acid, 2-hydroxyheptanoic acid, 2-hydroxyoctanoic acid, 2-hydroxynonanoic acid, 2-hydroxydecanoic acid, 2-hydroxyundecanoic acid, 2-hydroxydodecanoic acid, 2-hydroxytetradecanoic acid, 2-hydroxyhexadecanoic acid, 2-hydroxyoctadecanoic acid, 2-hydroxytetraecosanoic acid, 2-hydroxyeicosanoic acid, benzilic acid, benzene-1,4-di(3-methylidene-10-camphorsulfonic acid)phenyllactic acid, gluconic acid, galacturonic acid, aleuritic acid, ribonic acid, tartronic acid, tartaric acid, malic acid, fumaric acid, their derivatives, betaine, creatine, or a mixture thereof.

20. The composition of anyone of claims 17 to 19, wherein the active agent is present in a concentration ranging from 0.01 to 20% of the total weight of the composition.

21. The composition of claim 20, which comprises at least one lipophilic or hydrophilic adjuvant selected from the group consisting of preservatives, antioxidants, sequestering agents, solvents, fragrances, fillers, screening agents, odor absorbers, coloring materials, and lipid vesicles.

22. A method of treating the skin and/or mucous membranes and/or keratinous fibers, comprising applying an effective amount of the composition of anyone of claims 13 to 21 to the skin and/or mucous membranes and/or keratinous fibers.

23. A method of conditioning hair, said method comprising applying an effective amount for providing a hair care effect on the hair of the composition of anyone of claims 13 to 21 to the hair.

24. A process for preparing an emulsion according to anyone of claims 1 to 12, **characterized by** (a) providing all components in a container and (b) homogenize the mixture at a temperature from about 20°C to about 90°C for about 1 to about 6 minutes at about 4,000 to about 20,000 rpm.
